# FASCICULE DE BREVET EUROPEEN

(11) **EP 2 007 394 B1**
(45) Date de publication et mention de la délivrance du brevet: **10.10.2012**
(21) Numéro de dépôt: 07726759.9
(22) Date de dépôt: 09.03.2007
(51) Int. Cl.: A61K 45/06, A61K 31/5415, A61P 19/02, A61P 31/12

(54) **NOUVELLE UTILISATION D'AGENTS ANTIHISTAMINIQUES POUR LE TRAITEMENT PREVENTIF OU PRECOCE DE SYNDROMES INFLAMMATOIRES, EN PARTICULIER CEUX DECLENCHES PAR LES TOGAVIRUS.**
NEUE VERWENDUNG VON ANTIHISTAMINIKA ZUR PRÄVENTIVEN ODER FRÜHZEITIGEN BEHANDLUNG VON ENTZÜNDLICHEN SYNDROMEN, INSBESONDERE AUSGELÖST DURCH TOGAVIREN
NOVEL USE OF ANTIHISTAMINE AGENTS FOR THE PREVENTIVE OR EARLY TREATMENT OF INFLAMMATORY SYNDROMES, IN PARTICULAR THOSE TRIGGERED BY TOGAVIRUSES

(30) Priorité: 09.03.2006 FR 0602087
(43) Date de publication de la demande: 31.12.2008
(73) Titulaire: PIERRE FABRE MEDICAMENT, 92100 Boulogne-Billancourt (FR)
(72) Inventeur: DEREGNAUCOURT, Jean, F-75014 Paris (FR); ANDRE, Etienne, F-38170 Seyssinet-pariset (FR); TISNE-VERSAILLES, Jacky, F-81100 Castres (FR)
(74) Mandataire: Ahner, Francis
(86) Numéro de dépôt international: PCT/EP2007/052237
(87) Numéro de publication internationale: WO 2007/101884

(56) Documents cités:
- WO-A-96/04787
- FR-A1- 2 802 101
- US-A- 3 726 976
- DATABASE EPODOC EUROPEAN PATENT OFFICE, THE HAGUE, NL; 23 mars 1992 (1992-03-23), XP002405761 & JP 04 089428 A (KAO CORP) 23 mars 1992 (1992-03-23)
- "Pharyngeal composition contains dimemorfan, and one or more of ambroxol, bromhexine, guaifenesin, chlorpheniramine, carbinoxamine and mequitazine, for preventing inflammation" DERWENT, 5 juin 2001 (2001-06-05), XP002300900
- TOOVEY S ET AL: "Falciparum malaria presenting with pruritic rashes" TRAVEL MEDICINE AND INFECTIOUS DISEASE 2004 UNITED STATES, vol. 2, no. 3-4, 2004, pages 189-191, XP002403395 ISSN: 1477-8939
- SUHRBIER A ET AL: "Clinical and pathologic aspects of arthritis due to Ross River virus and other alphaviruses" CURRENT OPINION IN RHEUMATOLOGY 2004 UNITED STATES, vol. 16, no. 4, 2004, pages 374-379, XP009073786 ISSN: 1040-8711
- STOCKS N ET AL: "Ross River virus infection. Diagnosis and treatment by general practitioners in South Australia." AUSTRALIAN FAMILY PHYSICIAN. JUN 1997, vol. 26, no. 6, juin 1997 (1997-06), pages 710-717, XP009073788 ISSN: 0300-8495
- PAGANIN F ET AL: "Chikungunya on Reunion Island: Chronicle of an epidemic foretold" PRESSE MEDICALE 2006 FRANCE, vol. 35, no. 4 II, 2006, pages 641-646, XP009073781 ISSN: 0755-4982

## Description

La présente invention concerne une nouvelle utilisation d'agents antihistaminiques H1 de type phénothiazinique pour la préparation d'un médicament destiné au traitement préventif ou précoce de l'arthrite d'origine virale et en particulier de l'arthrite des articulations distales.

De manière générale, à la suite d'une agression tissulaire localisée ou diffuse, que ce soit un traumatisme ou une infection, une cascade complexe de réactions est élaborée par l'organisme pour éliminer l'agent infectieux et mettre en oeuvre un processus de réparation. Cette séquence d'événements s'appelle la réaction inflammatoire et associe des phénomènes locaux et l'élaboration de signaux systémiques. Le déclenchement de la cascade de réactions inflammatoires peut avoir un grand nombre d'origines : bactérienne, virale, parasitaire, mycotique, tumorale, traumatique, physique, et/ou encore immunologique. Les anomalies biologiques et cliniques systémiques constatées à cette occasion constituent le syndrome inflammatoire. La production de cytokines par les macrophages joue un rôle central dans l'orchestration des mécanismes qui contribuent à la mise en place d'une réaction inflammatoire. La résultante de la circulation systémique des trois principales cytokines de l'inflammation (IL1, IL6, et TNF α) dont la production est excessive lors de l'exacerbation de la réponse inflammatoire constitue le syndrome inflammatoire.

La présente invention concerne plus particulièrement les syndromes inflammatoires dans lesquels on observe majoritairement des lésions des tissus articulaires. L'arthrite rhumatoïde en est un exemple. De façon marginale, d'autres tissus peuvent être atteints, par exemple le coeur, les poumons, les muscles squelettiques, les vaisseaux périphériques, ou le système nerveux central. L'étiologie de cette affection, bien que largement étudiée, reste obscure. Les facteurs déclenchants comprennent les agents infectieux, l'hérédité, les facteurs alimentaires, mais aussi les réactions auto-immunes. Dans ce dernier cas, la réponse à l'agression externe a comme point de départ une réaction antigène/anticorps qui conduit à un complexe immun qui est le facteur déclenchant d'une suite de phénomènes biologiques. En marge de cette cascade de phénomènes destinés à combattre les agressions, les complexes immuns présentent un potentiel pathologique. Alors que les anticorps ont deux sites de combinaison avec les antigènes dans le cas des immunoglobulines IgG ou cinq sites de combinaison dans le cas des immunoglobulines IgM, les antigènes sont multivalents. Les complexes ainsi formés lors de cette association antigène/anticorps possèdent des tailles et des solubilités différentes, et leur potentiel pathologique dépend de leurs proportions relatives. Les mécanismes par lesquels les complexes immuns peuvent conduire à des lésions au niveau de certains tissus ont fait l'objet de nombreuses études. En particulier, il a été mis en évidence qu'ils pouvaient influencer la libération d'agents vasoactifs des plaquettes, des neutrophiles et des mastocytes tant par action dépendante du complément qu'en dehors. Parmi ces agents, les amines vasoactives augmentent la perméabilité vasculaire, ce qui provoque l'extravasasion et le dépôt d'une grande quantité de complexes antigènes/anticorps au niveau des membranes peri et extravasculaires. Un processus inflammatoire se déclenche alors dans le cas où des leucocytes polymorphonucléaires sont recrutés et s'accumulent. Des lésions peuvent également se produire si les complexes antigènes/anticorps précipitent. L'activation de la séquence du complément qui en résulte conduit, après phagocytose du complexe, à la libération de cytokines et d'enzymes lysosomiales à partir des leucocytes polymorphonucléaires et des monocytes. Ces enzymes peuvent être à l'origine d'une augmentation de l'inflammation et provoquer des lésions au niveau de différents tissus.

En dehors de l'arthrite rhumatoïde, inflammation chronique, il existe des inflammations articulaires provenant d'infections bactériennes, mycosiques ou virales. Les arthrites aiguës infectieuses sont en générale d'origines bactériennes ou virales. Dans le cas des arthrites bactériennes, les germes en cause sont les gonocoques, les staphylocoques, les streptocoques, les pneumocoques, heamophilus, spirochètes. L'arthrite non gonococcique est habituellement causée par le staphylococcus aureus, par des spirochètes, par des streptocoques ou d'autres germes gram-. Dans le cas d'arthrites virales aiguës, les principales causes identifiées en sont le parvovirus B19, les flavivirus, les virus des hépatites B et C, le virus de la rubéole, de la rougeole, les togavirus (incluant les alphavirus). D'autres pathologies sont aussi associées à des anthralgies et à de l'arthrite. C'est le cas de la varicelle, des oreillons, des maladies à adénovirus, des maladies à virus coxackie A9, B2, B3, B4, et B6, et de la mononucléose de l'Epstein-Barr.

Dans toutes ces situations, l'infection entraîne au niveau des articulations, une réaction inflammatoire suivant un processus analogue à celui mentionné ci-dessus.

Les manifestations cliniques de l'arthrite infectieuse au niveau des articulations sont la douleur et la tuméfaction au point d'inflammation. Elles touchent parfois plusieurs articulations. Ces manifestations surviennent en général soudainement, et s'accompagnent de fièvres et de frissons. Dans le cas d'une infection d'origine virale, des sensations de malaise généralisé viennent s'ajouter.

Les traitements disponibles actuellement font appel à des anti-inflammatoires non stéroïdiens (AINS), notamment pour soulager la douleur, la tuméfaction, et la raideur causées par l'arthrite infectieuse, mais ils ne contribuent pas à prévenir les lésions articulaires et ses conséquences. A plus fortes doses, les AINS concourent également à soulager l'inflammation. Dans le cas des arthrites infectieuses d'origine bactérienne, des antibiotiques sont utilisés, mais dans le cas des arthrites d'origine virale aucun médicament n'est en général prescrit dans le but de traiter la cause de la pathologie. La plupart du temps, l'infection disparaît spontanément.

Il existe donc un besoin de substances pharmacologiquement actives, et capables d'agir de manière précoce, voire préventive pour éviter les conséquences néfastes du processus inflammatoire mis en place lors d'infections bactériennes ou virales au niveau des articulations.

La Demanderesse a mis en évidence que certains agents anti-histaminiques permettraient d'atteindre ce but.

Les anti-histaminiques sont des substances s'opposant à l'activité de l'histamine sur ses récepteurs spécifiques et, en conséquence, s'opposant aux diverses réactions qu'elle provoque. Ces inhibiteurs n'agissent sélectivement, voire spécifiquement, que sur un sous-type de récepteur, d'où plusieurs classes d'antihistaminiques: les antihistaminiques H1, les antihistaminiques H2, les antihistaminiques H3.

Les antihistaminiques H2 sont de puissants inhibiteurs de la sécrétion gastrique, utilisés dans la maladie ulcéreuse et secondairement contre le reflux gastro-oesophagien et le pyrosis.

Les antihistaminiques H1 agissent contre les effets vasculaires de l'histamine et les réactions d'hypersensibilité. Ce sont des médicaments à visée symptomatique d'un certain nombre de manifestations allergiques notamment au niveau des muqueuses et de la peau. Ils sont prescrits dans les principales indications telles que les rhinites allergiques, les dermatoses allergiques (formes papulo-oedémateuse), les oedèmes de Quincke, contre les effets secondaires des traitements de désensibilisation, dans le traitement de la maladie sérique et dans les réactions allergiques aux médicaments.

Ces anti-H1 sont groupés selon une classification qui s'inspire de leur structure chimique. On dénombre ainsi cinq classes principales :
- Les éthylène diamines,
- Les benzhydryl alkylamines,
- Les benzhydryl pipérazines,
- Les pyridylbenzyl alkylamines, et
- Les phénothiazinyl alkylamines.

Parmi ces familles, les phénothiazinyl alkylamines sont des antihistaminiques H1 puissants mais possèdent parfois des propriétés vis à vis de récepteurs d'autres amines biogènes.

La présente invention concerne donc l'utilisation d'au moins un agent antihistaminique H1 de type phénothiazinique ou de l'un de ses sels d'addition à un acide ou à une base pharmaceutiquement acceptable, pour la préparation d'un médicament destiné au traitement préventif ou précoce de l'arthrite d'origine virale et en particulier de l'arthrite des articulations distales.

Un aspect avantageux de l'invention concerne l'utilisation d'au moins un agent antihistaminique H1 de type phénothiazinique pour la préparation d'un médicament destiné au traitement préventif ou précoce de syndromes inflammatoires d'origine virale déclenché par un alphavirus, en particulier le virus Chikungunya, le virus River Ross et le virus Barmah Forest.

Le Chikungunya est une maladie infectieuse tropicale due à un arbovirus de la famille des Togaviridae (alphavirus), transmise par des moustiques du genre Aedes. L'aedes se reproduit facilement dans de l'eau douce, stagnante, non croupie et à l'ombre. Les gîtes larvaires sont par exemple les récipients abandonnés, les bâches de piscine, les pneus abandonnés....

Ces espèces sont également impliquées dans la transmission d'autre arbovirus tels que les virus de la dengue, de la fièvre jaune etc...

La contamination d'un homme sain est provoquée par la salive des moustiques femelles qui ont été infectées. La transmission du virus d'un humain malade à un moustique se fait par le sang aspiré lors de la piqûre, le sang traverse ensuite la barrière stomacale du moustique pour passer dans les glandes salivaires. L'incubation de la maladie dure de 4 à 7 jours.

La maladie se déclare par une très forte fièvre suivie d'un érythème et de courbatures très douloureuses, agissant principalement sur les petites articulations des mains poignets, chevilles et pieds. La douleur est de type inflammatoire, forte le matin et améliorée par des mouvements doux. Un gonflement des articulations peut se produire.

Ces fortes douleurs articulaires sont associées à une raideur (allure courbée des patients). Les manifestations cutanées consistent en éruptions papulaires ou maculopapulaires atteignant le tronc et les membres, accompagnées de prurit et d'irritation. Dans les cas les plus sévères ces symptômes peuvent être accompagnés de maux de tête de nausées et de vomissements. Il existe cependant des formes asymptomatiques de la maladie.

A ce jour aucun médicament n'a été mis au point, et aucun vaccin n'est finalisé. Il n'existe en outre aucun traitement virucide. Le traitement est donc purement symptomatique, et consiste à faire tomber la fièvre et réduire la douleur.

Parmi les antihistaminiques H1 de type phénothiazinique convenant à la mise en oeuvre de la présente invention, on choisira de préférence l'aminopromazine, la chlopromazine, la cyamémazine, l'isoprométhazine, la lévopromazine, la méquitazine, la pipotiazine, la prométhazine, la thioridazine, et le méthylsulfate de thiazinamium. Une phénothiazine convenant particulièrement bien à la nouvelle utilisation selon la présente invention est la méquitazine.

Les principes actifs ci-dessus ainsi que ceux mentionnés dans la suite, sont nommés par leur Dénomination Commune Internationale (DCI).

La Demanderesse a également mis en évidence que le traitement des inflammations articulaires selon la présente invention pouvait être considérablement amélioré, lorsque les composés mis en oeuvre présentent, outre leur activité antihistaminique, une composante antisérotonine. En effet la sérotonine est une autre amine vasoactive mise en jeu lors du processus inflammatoire, et influençant la perméabilité vasculaire.

Selon cet autre aspect, l'invention concerne donc l'utilisation d'au moins un agent antihistaminique H1 de type phénothiazinique pour la préparation d'un médicament destiné au traitement préventif ou précoce de syndromes inflammatoires d'origine virale tels que définis ci-dessus, et plus particulièrement ceux déclenchés par un alphavirus, caractérisée en ce que ledit agent antihistaminique H1 de type phénothiazinique présente également une composante antisérotonine. On choisira de préférence un antihistaminique H1 de type phénothiazinique possédant une composante antisérotonine, et de manière avantageuse un composé choisi parmi l'alimémazine, la chloropromazine, la cyamémazine, la lévopromazine, la méquitazine, la pipotiazine, la thioridazine.

Pour la mise en oeuvre de la présente invention, les composés peuvent présenter un ou plusieurs centres asymétriques dans leur structure chimique. Dans ce cas l'utilisation selon l'invention concerne aussi bien les composés sous forme de mélange racémique, mais aussi sous la forme de d'un seul des éventuels épimères, diastéréoisomères, ou énantiomères.

L'invention concerne aussi l'utilisation des composés selon la présente invention sous la forme de leurs sels d'addition avec un acide ou une base pharmaceutiquement acceptable.

Parmi les acides pharmaceutiquement acceptables, on peut citer de façon non limitative les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthansulfonique, camphorique, axalique ...

Parmi les bases pharmaceutiquement acceptables, on peut citer à titre non limitatif, l'hydroxyde de sodium, l'hydroxyde de potassium, la triéthylamine, la tertbutylamine, ...

La présente invention concerne l'utilisation telle que définie ci-dessus, caractérisée en ce que le médicament se présente sous la forme d'une composition pharmaceutique adaptée à toute forme d'administration habituellement envisagée. Ces compositions pharmaceutiques contiennent au moins un composé antihistaminique tel que défini précédemment, utilisé dans le cadre de la présente invention, seul ou en combinaison avec un ou plusieurs excipients pharmaceutiquement acceptable.

Parmi les formes pharmaceutiques, on peut citer plus particulièrement celles qui conviennent à l'administration orale, parentérale, per- ou transcutanée, rectales, linguales ou sublinguales, et notamment les comprimés simples ou dragéifiés, les comprimés sublinguaux, les sachets, les paquets, les gélules, les tablettes, les suppositoires, les crèmes, les pommades, les ampoules buvables, les ampoules et/ou solutés injectables, les sirops. La posologie varie avec l'âge et le poids du patient, la voie d'administration, ou les éventuels traitements associés.

Dans un aspect préféré de l'invention, le médicament se présente sous la forme d'un comprimé dont la dose en agent antihistaminique tel que défini précédemment est comprise entre 0,5 mg et 200 mg, et plus particulièrement entre 5mg et 100 mg. De préférence cet agent antihistaminique est la méquitazine.

Dans un autre aspect avantageux de l'invention, le médicament se présente sous la forme d'un sirop dont la concentration en agent antihistaminique tel que défini précédemment est comprise entre 0,01 % et 1 %, c'est-à-dire varie entre 0,1 mg/ml, et 10 mg/ml. De préférence cet agent antihistaminique est la méquitazine.

Le médicament peut aussi se présenter sous la forme de gélules dont le dosage en agent antihistaminique tel que défini précédemment varie entre 0,1 mg et 15 mg, sous la forme de solutions buvables dont la concentration en agent antihistaminique tel que défini précédemment varie entre 1 mg/ml et 70 mg/ml, ou sous la forme de soluté injectable, notamment dans des ampoules de 2 ml, dont la concentration en agent antihistaminique tel que défini précédemment varie entre 5 mg/ml et 50 mg/ml.

Dans un autre aspect, la présente invention concerne un produit de combinaison d'au moins un agent antihistaminique H1 de type phénothiazinique et d'au moins un agent antisérotonine pour son utilisation simultanée, séparée ou étalée dans le temps, en thérapie préventive ou précoce des syndromes inflammatoires d'origine virale, et plus particulièrement déclenchés par un alphavirus, et plus spécifiquement le virus chikungunya.

Le choix du ou des agents antisérotonines à associer avec le ou les agents antihistamines dans le produit selon l'invention se fera en fonction de l'importance du caractère antagoniste du composé antihistamine. Ainsi l'action simultanée sur les deux type de récepteurs histaminiques et sérotoninergiques pourra être modulée par l'association de deux agents adéquats.

De manière avantageuse dans le produit de combinaison selon l'invention, 1 antihistaminique H1 de phénothiazinique est préférentiellement choisi parmi, l'alimémazine, la chlopromazine, la cyamémazine, l'isoprométhazine, la lévopromazine, la méquitazine, la pipotiazine, la prométhazine, la thioridazine, et le méthylsulfate de thiazinamium. Une phénothiazine convenant particulièrement bien est la méquitazine.

Parmi les composés antisérotonine convenant à la mise en oeuvre de la présente invention, on peut citer les antagonistes des récepteurs 5-HT_{2A}, 5-HT_{2C}, et/ou 5-HT₃. On choisira par exemple l'amixétrine, le bencyclane, la chlorphénoxamine, le chlorprothixène, la dimétotiazine, le fenpentadiol, la médifoxamine, la methdilazine, la périzacine, l'oxaflumazine, la rispéridone, la ritansérine, le seroquel, l'olanzapine, la mesulergine, la miansérine, la ritansérine, la granisetron, la dihydroergotamine, le dolasetron, l'ondensetron, l'ergotamine, la flopropione, la kétansérine, la méthylergométrine, la méthysergide, le naftidrofuryl, la tolazoline et le tropisetron.

La présente invention est illustrée par les exemples suivants qui ne la limitent en aucune façon. Ainsi l'utilisation des agents antihistaminiques selon l'invention peut être démontrée dans le cadre d'études observationnelles et d'essais cliniques menés selon des techniques et des méthodologies standard.

### Exemple 1 : Comprimé à base de méquitazine, dosés à 5 ou 10 mg.

| | |
|---|---|
| Méquitazine (DCI) | 5 mg (ou 10 mg) |
| Lactose monohydraté | qs |
| Amidon de maïs | qs |
| Gomme arabique | qs |
| Silice colloïdale anhydre | qs |
| Talc | qs |
| Carboxyméthylamidon sodique | qs |
| Stéarate de magnésium | qs |

### Exemple 2 : Sirop à base de méquitazine.

| | |
|---|---|
| Méquitazine (DCI) | 1,25 mg / p c mesure* |
| Acide ascorbique | qs |
| Essence soluble de mandarine | qs |
| Saccharose | qs |
| Eau purifiée | qs |
| Conservateurs | qs |

| | |
|---|---|
| (* : p c mesure = par cuillère mesure de 2,5 ml de contenance) | |

### Exemple 3 : 1 Effet de la méquitazine sur le traitement préventif et précoce des syndromes inflammatoires ayant pour origine le virus du Chikungunya - Résultats d'une étude observationnelle

### Protocole

104 patients ont été inclus dans cette étude observationnelle confiée à un nombre limité de médecins tenus au secret : âge moyen 39,6 ans, 61,9% de femmes.

Lors de l'inclusion à J0 les patients étaient légèrement fiévreux avec une température moyenne de 38,4 °C.

Les patients consultaient dans des délais divers après l'apparition de l'hyperthermie mais en moyenne 1,1 jours après cette apparition, phase précédent l'apparition de l'arthralgie.

Lors de cette première visite, les articulations les plus douloureuses étaient les doigts de la main, le poignet et la cheville.

Lors de cette première visite à J0, les médecins donnaient aux patients de la méquitazine, éventuellement associée à un anti-inflammatoire.

Un questionnaire était remis à chaque patient et rempli avec lui.

Ce questionnaire inclut le contenu de 2 questionnaires classiques :
- Un questionnaire HAQ* (Health Assessment Questionnaire) qui permet de mesurer la capacité fonctionnelle des patients (indice fonctionnel) c'est-à-dire l'handicap généré par la pathologie ;
- Un questionnaire SF12** qui permet d'évaluer la qualité de la vie du patient au moyen de l'échelle SF12.

A J7 et J14, le patient remplissait à nouveau ces questionnaires.

A J28, lors d'une nouvelle visite chez le médecin, ces questionnaires étaient à nouveau remis à jour.
* HAQ: Bruce B and Fries J. The Stanford Health Assessment Questionnaire (HAQ):A review of its history, issues, progress and documentation. J Rheumatol 2003;30(1):167-78
** SF12: Ware J Jr, Kosinski M, Keller SD. A 12-Item Short-Form Health Survey: construction of scales and preliminary tests of reliability and validity. Med Care 1996; 34:220-33

### Résultats

L'analyse statistique de ces questionnaires fait apparaître les résultats suivants :
- Mesure du handicap généré par la pathologie (indice fonctionnel HAQ) : L'administration de méquitazine entraîne une amélioration statistiquement significative à J14 et J28
- Entre J0 et J7, l'administration de méquitazine entraîne une diminution significative du nombre d'articulations douloureuses, cette diminution se pérennisant dans le temps.
- Plus le patient consulte tôt et donc plus l'administration de méquitazine est précoce, plus le rétablissement au niveau physique (échelle SF12 et test HAQ) est important et rapide
- L'adjonction à la méquitazine d'un anti-inflammatoire n'influence pas le score physique SF12 qui mesure la qualité de vie.

## Revendications

1. Utilisation d'au moins un antihistaminique H1 de type phénothiazinique, ou l'un de ses sels d'addition à un acide ou à une base pharmaceutiquement acceptable, pour la préparation d'un médicament destiné au traitement préventif ou précoce de l'arthrite d'origine virale et en particulier de l'arthrite des articulations distales.

2. Utilisation selon la revendication 1, **caractérisée en ce que** l'arthrite d'origine virale est déclenchée par un alphavirus, en particulier le virus Chikungunya, le virus Ross River ou le virus Barmah Forest.

3. Utilisation selon l'une des revendications 1 et 2, **caractérisée en ce que** ledit antihistaminique H1 de type phénothiazinique présente également une composante antisérotonine.

4. Utilisation selon l'une des revendications 1 à 3, **caractérisée en ce que** ledit agent antihistaminique H1 de type phénothiazinique est choisi parmi l'alimémazine, l'aminopromazine, la chlopromazine, la cyamémazine, l'isoprométhazine, la lévopromazine, la méquitazine, la pipotiazine, la prométhazine, la thioridazine, et le méthylsulfate de thiazinamium.

5. Utilisation selon l'une des revendications 1 à 4, **caractérisée en ce que** ledit médicament se présente sous la forme d'un comprimé dont la dose en agent antihistaminique H1 de type phénothiazinique est comprise entre 0,5 mg et 200 mg.

6. Utilisation selon la revendication 5, **caractérisée en ce que** l'antihistaminique H1 de type phénothiazinique est la méquitazine.

7. Utilisation selon l'une des revendications 1 à 4, **caractérisée en ce que** ledit médicament se présente sous la forme d'un sirop dont la concentration en agent antihistaminique H1 de type phénothiazinique est comprise entre 0,01 et 1 %, en poids par volume.

8. Utilisation selon la revendication 7, **caractérisée en ce que** l'agent antihistaminique H1 de type phénothiazinique est la méquitazine.

9. Produit de combinaison d'au moins un agent antihistaminique H1 de type phénothiazinique et d'au moins un agent antisérotonine pour son utilisation simultanée, séparée ou étalée dans le temps, en thérapie préventive ou précoce de l'arthrite d'origine virale, et en particulier de l'arthrite des articulations distales.

10. Produit selon la revendication 9, **caractérisé en ce que** l'arthrite d'origine virale est déclenchée par un alphavirus, en particulier le virus Chikungunya, le virus Ross River ou le virus Barmah.Forest

11. Produit selon l'une des revendications 9 à 10, **caractérisé en ce que** l'agent antihistaminique H1 de type phénothiazinique est choisi parmi l'alimémazine, l'aminopromazine, la chlopromazine, la cyamémazine, l'isoprométhazine, la lévopromazine, la méquitazine, la pipotiazine, la prométhazine, la thioridazine, et le méthylsulfate de thiazinamium.

12. Produit selon l'une des revendications 9 à 11, **caractérisé en ce que** l'agent antisérotonine est choisi parmi l'amixétrine, le bencyclane, la chlorphénoxamine, le chlorprothixène, la dimétotiazine, le fenpentadiol, la médifoxamine, la méthdilazine, la périzacine, l'oxaflumazine, la rispéridone, la ritansérine, le séroquel, l'olanzapine, la mésulergine, la miansérine, la ritansérine, la granisétron, la dihydroergotamine, le dolasétron, l'ondensetron, l'ergotamine, la flopropione, la kétansérine, la méthylergométrine, le méthysergide, le naftidrofuryl, la tolazoline et le tropisétron.

## Claims

1. Use of at least one H1-antihistamine of phenothiazine type, or an addition salt thereof with a pharmaceutically acceptable acid or base, to prepare a medicinal product intended for the preventive or early treatment of viral arthritis, in particular arthritis of the distal joints.

2. Use according to claim 1, **characterized in that** the viral arthritis is triggered by an alphavirus, in particular the Chikungunya virus, Ross River virus or Barmah Forest virus.

3. Use according to either of claims 1 and 2, **characterized in that** said phenothiazine H1-antihistamine also contains an antiserotonin component.

4. Use according to any of claims 1 to 3, **characterized in that** said H1-antihistamine of phenothiazine type is chosen from among alimemazine, aminopromazine, chlorpromazine, cyamemazine, isopromethazine, levopromazine, mequitazine, pipotiazine, promethazine, thioridazine and thiazinamium methylsulfate.

5. Use according to any of claims 1 to 4, **characterized in that** said medicinal product is in the form of a tablet whose dose of H1-antihistamine of phenothiazine type ranges from 0.5 mg to 200 mg.

6. Use according to claim 5, **characterized in that** the H1-antihistamine of phenothiazine type is mequitazine.

7. Use according to any of claims 1 to 4, **characterized in that** said medicinal product is in the form of a syrup whose concentration of H1-antihistamine of phenothiazine type lies between 0.01 and 1 weight-volume percentage.

8. Use according to claim 7, **characterized in that** the H1-antihistamine of phenothiazine is mequitazine.

9. Combination product of at least one phenothiazine H1-antihistamine and at least one antiserotonin agent for its simultaneous, separate or time-release use as preventive or early therapy for viral arthritis, in particular arthritis of the distal joints.

10. Product according to claim 9, **characterized in that** the viral arthritis is triggered by an alphavirus, in particular the Chikungunya virus, Ross River virus or Barmah Forest virus.

11. Product according to either of claims 9 and 10, **characterized in that** the H1-antihistamine of phenothiazine is chosen from among alimemazine, aminopromazine, chlorpromazine, cyamemazine, isopromethazine, levopromazine, mequitazine, pipotiazine, promethazine, thioridazine and thiazinamium methylsulfate.

12. Product according to any of claims 9 to 11, **characterized in that** the antiserotonin agent is chosen from among amixetrine, bencyclane, chlorphenoxamine, chlorprothixene, dimetotiazine, fenpentadiol, medifoxamine, methdilazine, perizacin, oxaflumazine, risperidon, ritanserin, seroquel, olanzapin, mesulergin, mianserin, ritanserin, granisetron, dihydroergotamine, dolasetron, ondensetron, ergotamine, flopropione, ketanserin, methylergometrin, methysergide, naftidrofuryl, tolazolin and tropisetron.

## Patentansprüche

1. Verwendung mindestens eines H1-Antihistaminikums vom Phenothiazin-Typ, oder eines von dessen pharmazeutisch akzeptablen Säure- oder Baseadditionssalzen, für die Herstellung eines Arzneimittels, das für die Präventiv- oder Frühbehandlung von Arthritis viralen Ursprungs, und insbesondere von Arthritis der distalen Gelenke, bestimmt ist.

2. Verwendung nach Anspruch 1, **dadurch gekennzeichnet, dass** die Arthritis viralen Ursprungs durch ein Alphavirus, insbesondere das Chikungunya-Virus, das Ross-River-Virus oder das Barmah-Forest-Virus, ausgelöst wurde.

3. Verwendung nach einem der Ansprüche 1 und 2, **dadurch gekennzeichnet, dass** das H1-Antihistaminikum vom Phenothiazin-Typ auch einen Antiserotonin-Baustein aufweist.

4. Verwendung nach einem der Ansprüche 1 bis 3, **dadurch gekennzeichnet, dass** der H1-Antihistaminikum-Wirkstoff vom Phenothiazin-Typ ausgewählt ist aus Alimemazin, Aminopromazin, Chlorpromazin, Cyamemazin, Isopromethazin, Levopromazin, Mequitazin, Pipotiazin, Promethazin, Thioridazin und Thiazinamiummethylsulfat.

5. Verwendung nach einem der Ansprüche 1 bis 4, **dadurch gekennzeichnet, dass** das Arzneimittel in Form einer Tablette vorliegt, deren Dosis an H1-Antihistaminikum-Wirkstoff vom Phenothiazin-Typ zwischen 0,5 mg und 200 mg beträgt.

6. Verwendung nach Anspruch 5, **dadurch gekennzeichnet, dass** das H1-Antihistaminikum vom Phenothiazin-Typ Mequitazin ist.

7. Verwendung nach einem der Ansprüche 1 bis 4, **dadurch gekennzeichnet, dass** das Arzneimittel in Form eines Sirups vorliegt, dessen Konzentration an H1-Antihistaminikum-Wirkstoff vom Phenothiazin-Typ zwischen 0,01 und 1 Volumen-% beträgt.

8. Verwendung nach Anspruch 7, **dadurch gekennzeichnet, dass** der H1-Antihistaminikum-Wirkstoff vom Phenothiazin-Typ Mequitazin ist.

9. Kombinationsprodukt aus mindestens einem H1-Antihistaminikum-Wirkstoff vom Phenothiazin-Typ und mindestens einem Antiserotonin-Wirkstoff für die gleichzeitige, getrennte oder zeitversetzte Verwendung in der Präventiv- oder Frühtherapie von Arthritis viralen Ursprungs, und insbesondere von Arthritis der distalen Gelenke.

10. Produkt nach Anspruch 9, **dadurch gekennzeichnet, dass** die Arthritis viralen Ursprungs durch ein Alphavirus, insbesondere das Chikungunya-Virus, das Ross-River-Virus oder das Barmah-Forest-Virus, ausgelöst wurde.

11. Produkt nach einem der Ansprüche 9 bis 10, **dadurch gekennzeichnet, dass** der H1-Antihistaminikum-Wirkstoff vom Phenothiazin-Typ ausgewählt ist aus Alimemazin, Aminopromazin, Chlorpromazin, Cyamemazin, Isopromethazin, Levopromazin, Mequitazin, Pipotiazin, Promethazin, Thioridazin und Thiazinamiummethylsulfat.

12. Produkt nach einem der Ansprüche 9 bis 11, **dadurch gekennzeichnet, dass** der Antiserotonin-Wirkstoff ausgewählt ist aus Amixetrin, Bencyclan, Chlorphenoxamin, Chlorprothixen, Dimetotiazin, Fenpentadiol, Medifoxamin, Methdilazin, Periciazin, Oxaflumazin, Risperidon, Ritanserin, Seroquel, Olanzapin, Mesulergin, Mianserin, Ritanserin, Granisetron, Dihydroergotamin, Dolasetron, Ondansetron, Ergotamin, Flopropion, Ketanserin, Methylergometrin, Methysergid, Naftidrofuryl, Tolazolin und Tropisetron.
